# EUROPEAN PATENT APPLICATION

(11) **EP 4 563 593 A1**
(43) Date of publication of application: **04.06.2025**
(21) Application number: 23213245.6
(22) Date of filing: 30.11.2023
(51) Int. Cl.: C07K 14/415, C12N 9/22, C12N 15/82

(54) **INCREASING THE PHOTOSYNTHESIS OF A PLANT, PLANT PART, OR PLANT CELL**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a method for increasing the photosynthesis of a plant, plant part, or plant cell by increasing the expression of the squamosa promoter binding protein-like gene 9 (SPL9 gene) in the plant, plant part, or plant cell and/or by increasing the level of the squamosa promoter binding protein-like protein 9 (SPL9 protein) in the plant, plant part, or plant cell.

## Description

The present invention relates to a method for increasing the photosynthesis of a plant, plant part, or plant cell by increasing the expression of the squamosa promoter binding protein-like gene 9 (SPL9 gene) in the plant, plant part, or plant cell and/or by increasing the level of the squamosa promoter binding protein-like protein 9 (SPL9 protein) in the plant, plant part, or plant cell.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Increased CO₂ in the atmosphere is such a serious problem for humankind that many research and development approaches are implemented to reduce CO₂ emissions. The majority of countries have issued laws and policies in response to this concern by requiring their industrial sectors to reduce greenhouse gas emissions, such as CO₂. One possible approach is biological CO₂ fixation using the photosynthetic function of plats or microorganisms, such as microalgae. Other studies have demonstrated CO₂ fixation using photo-bioreactors and raceway ponds. A comprehensive review of CO₂ fixation through biological processes is presented in Goli et al. (2016), Journal of Environmental Management, 183(1):41-58.

While some means and methods for CO₂ fixation through biological processes are already available there is an ongoing need for further and in particular effective means and methods. Such A comprehensive review of CO₂ fixation through biological processes are crucial for finally achieving the desired control of the emission of the greenhouse gas CO₂ with the ultimate aim of stopping global warming.

This need is addressed by the present invention.

Accordingly, the present invention relates in a first aspect to a method for increasing the photosynthesis of a plant, plant part, or plant cell by increasing the expression of the squamosa promoter binding protein-like gene 9 (SPL9 gene) in the plant, plant part, or plant cell and/or by increasing the level of the squamosa promoter binding protein-like protein 9 (SPL9 protein) in the plant, plant part, or plant cell.

The squamosa promoter binding protein-like gene 9 (*SPL9* gene; also known as *AtSPL9;* T24P15.11; T24P15_11) encodes a putative transcriptional regulator that is involved in the vegetative to reproductive phase transition and vegetative phase change in leaves. SPL activity nonautonomously inhibits initiation of new leaves at the shoot apical meristem; see Gene ID: 818820, version of 7-Sep-2023.

*SPL9* is a family member of the squamosa promoter-binding protein-Like (SPL) genes that are known to affect a broad range of plant biological processes and show potential application in crop improvement by genetic modification; see Ma et al. (2021), Front. Plant Sci., Volume 12. These processes include the timing of vegetative phase changes and floral induction, the rate of leaf initiation, shoot regeneration and branching, anthocyanin and trichome production, stress responses, carotenoid biosynthesis, and lateral root development. SPL proteins are characterized by the presence of a highly conserved SQUAMOSA-PROMOTER BINDING PROTEIN (SBP) domain which consists of approximately 78 amino acid residues containing a nuclear localization signal (NLS) motif and two Zn finger-like structural motifs. SPL genes regulate the transcription of downstream genes through the binding of the SBP domain to GTAC core motif, thereby participating in the regulation of plant growth and development. In addition, most SPL genes can be degraded by miRNAs and the miRNA Responsive Element (MRE) lie downstream of the conserved SBP domain. For instance, it is known that the microRNA156 (miR156) targets a series of SPL genes, including the SPL9 gene. Reduced miR156 levels resulted in elevated SPL9 RNA levels; see Wang et al. (2008), The Plant Cell, 20:1231-1243, Xu et al (2016), PLoS Genet., 19;12(8):e1006263 and Schwarz et al. (2008), Mol Biol, 67(1-2):183-95.

The plant, or the plant from which the plant part or plant cell is derived is not particularly limited as long as the plant is a photosynthetic plant. The plant cell can also be a group of plant cells. Preferred examples will be provided herein below.

Photosynthesis is the process by which plants use sunlight, water, and carbon dioxide to create oxygen and energy in the form of sugar. Plants are autotrophs, which means they produce their own food. They use the process of photosynthesis to transform water, sunlight, and carbon dioxide into oxygen, and simple sugars that the plant uses as fuel. The sugars produced by photosynthesis are used both as an energy source by the plant and to produce the complex organic polymers that make up the plant.

In particular transformation of carbon dioxide (CO₂) into oxygen (O₂) by plants is of high commercial interest. As explained above CO₂ is the earth's most important greenhouse gas and causes global warming. Unlike oxygen or nitrogen (which make up most of our atmosphere), greenhouse gases absorb heat radiating from the Earth's surface and re-release it in all directions; including back towards the earth's surface. It follows that increasing the photosynthesis of a plant can help to transform atmospheric CO₂ into O₂, thereby reducing the atmospheric CO₂ content and helping prevent global warming.

In this connection the term "increasing the photosynthesis of a plant" preferably means that a plant, plant part, or plant cell wherein the expression of the SPL9 gene and/or the level of the SPL9 protein has been increased is capable of transforming more atmospheric CO₂ into O₂ than a corresponding plant, plant part, or plant cell without this increase. In this connection it is preferred with increasing preference that by the method of the invention a plant, plant part, or plant cell is obtained that can transform at least 10%, at least 20%, at least 30%, at least 40%, at least 50%, at least 60%, at least 70%, at least 80%, at least 90% or at least 100% more atmospheric CO₂ into O₂ than a corresponding (e.g. wild-type or unmodified) plant, plant part, or plant without increased *SPL9* expression and/or SPL9 protein levels.

Means and methods for determining whether a plant, plant part or plant cell displays increased photosynthesis are known in the art. For example, and preferably, the photosynthetic capacity (Aₘₐₓ) may be determined, which is a measure of the maximum rate at which leaves are able to fix carbon during photosynthesis. It is typically measured as the amount of carbon dioxide that is fixed per meter squared per second, for example as µmol m⁻² sec⁻¹.

The increase of the expression of the *SPL9* gene and/or the level of the SPL9 protein in the plant, plant part, or plant cell is with increasing preference an at least 1.5-fold, at least 2-fold, at-least 3-fold, at least 4-fold and at least 5-fold higher expression and/or or protein level as compared to the corresponding wild-type or unmodified plant, plant part, or plant cell.

The means and methods to be used for increasing the expression of the *SPL9* gene in the plant, plant part, or plant cell and/or for increasing the level of the SPL9 protein in the plant, plant part, or plant cell are not particularly limited.

For increasing the expression of the *SPL9* gene in the plant, plant part, or plant cell, for example, a nucleic acid molecule encoding an *SPL9* gene in expressible form can be introduced into the plant, plant part, or plant cell. In this connection the term "in expressible form" means that when the nucleic acid molecule encoding an *SPL9* gene is present in the *SPL9* gene is expressed into *SPL9* mRNA and this mRNA is translated into protein. Accordingly, the term "expression" (or gene expression) designates the process by which information from a gene is used in the synthesis of a functional gene product, which product is in the present case a protein. Hence, expression comprises the steps of transcription of a gene into mRNA and the translation of the mRNA into protein.

Increasing the expression of the *SPL9* gene preferably results in the overexpression of the *SPL9* gene. Overexpression is the excessive or high expression of a gene. As will be further explained herein below, an increased expression or overexpression of the *SPL9* gene can be achieved, for example, by placing the gene under the control of a stronger promoter as compared to the promoter controlling the gene in nature or by increasing the gene copy number in the genome.

The nucleic acid molecule encoding an *SPL9* gene is preferably comprised in a vector or plasmid. Many plant expression vectors and plasmid are available, for example, those being based on the Ti plasmid of *Agrobacterium tumefaciens.* In the latter plasmid vectors, DNA to be inserted into plant genome is cloned into the T-DNA, a stretch of DNA flanked by a 25-bp direct repeat sequence at either end, and which can integrate into the plant genome. Introducing plasmid vectors into plant cells is a widely-used procedure. Plasmid delivery can be accomplished, for example, by particle bombardment, chemical transfection, or electroporation. The Ti plasmid of *Agrobacterium tumefaciens* can be a T-DNA binary vector system to introduce genes into the genome of plants; see Lee et al. (2008), Plant Physiol.; 146(2):325-332.

An SPL9 gene on a plasmid may be expressed extrachromosomally but plasmids can also be used for the integration of an SPL9 gene into the genome of plant, plant part or plan cell.

Hence, an SPL9 gene cam also be expressed intrachromosomally by introducing it into the genome of the plant, plant part or plan cell. Thereby the gene copy number in the genome is increased. While expression from plasmids and genomic expression both work, genomic expression of the enzymes is preferred since it expected to provide more expression of the SPL9 gene.

The SPL9 gene is preferably introduced into the genome of the plant, plant part or plant cell by a CRISPR-based strategy for targeted transgene knock-in (covering both homology-dependent and homology-independent approaches). CRISPR may be combined with homology-directed repair (HDR), synthesis-dependent strand annealing (SDSA), microhomology-mediated end joining (MMEJ), and homology-mediated end joining (HMEJ) pathways for a homology-dependent strategy and alternative DNA repair pathways such as non-homologous end joining (NHEJ), base excision repair (BER), and mismatch repair (MMR) for a homology-independent strategy; see Lau et al. (2020), Fac Rev. 2020; 9: 20.

A CRISPR homology-directed repair (HDR)-based knock-in is preferred.

The SPL9 gene is preferably expressed under the control of a strong constitutive promoter. Placing a gene under the control of strong constitutively active promoter leads to the robust overexpression of the gene. Examples of strong constitutive promoters are the PGI promoter (preferably the PGI-20 promoter as used in the appended examples) and the T7 promoter.

An increase of the expression of the *SPL9* gene in the plant, plant part, or plant cell may also be achieved by introducing a mutation into the promoter region of the endogenous *SPL9* gene of the plant, plant part, or plant cell. The mutation has to result in a stronger promoter activity and therefore also in higher expression levels.

Finally, and as already discussed above an increase of the expression of the *SPL9* gene in the plant, plant part, or plant cell may also be achieved by reducing miRNA (preferably miR156) expression levels of those miRNAs that negatively regulate *SPL9* (e.g. by small molecule, an aptamer, a siRNA, a shRNA, a miRNA, a ribozyme, an antisense nucleic acid molecule, a CRISPR-based construct (such as a CRISPR-Cas9-based construct, or a CRISPR-Cpf1-based construct), a meganuclease, a zinc finger nuclease, and a transcription activator-like (TAL) effector (TALE) nuclease) and/or by mutating the miRNA Responsive Element (MRE) lying downstream of the conserved SBP domain of SPL9, so that miRNAs (preferably miR156) can no longer bind. This latter approach of mutating the miRNA Responsive Element (MRE) is illustrated by the appended examples. As also discussed above, most *SPL* genes are targeted by miRNAs and miR156 is known to degrade *SPL9* transcripts.

Related to the above, it is also possible to mutate the miRNA (preferably miR156), so that it can no longer bind to the MRE of *SPL9.*

The miRNA Responsive Element (MRE) or miRNA is preferably mutated by the CRISPR technology.

The above-discussed means and methods to increase the expression of the *SPL9* gene in the plant, plant part, or plant cell also result in increased levels of the SPL9 protein, because the expressed *SPL9* mRNA is translated into SPL9 protein.

It is also possible to selectively increase the levels of the SPL9 protein in plant, plant part, or plant cell by introducing recombinantly produced SPL9 protein into a plant, plant part, or plant cell. For example, particle guns can be used to introduce SPL9 protein into plant, plant part, or plant cell.

The plant, plant part, or plant cell as obtained by the method of the invention may be and is preferably a genetically engineered plant, plant part, or plant cell. The term "genetically engineered plant, plant part, or plant cell" as used herein designates a genetically engineered plant, plant part, or plant cell that has been genetically engineered such that the expression of the *SPL9* gene and/or the level of the SPL9 protein is increased as compared to a corresponding (wild-type or unmodified) plant, plant part, or plant cell that has been used to prepare the genetically engineered plant, plant part, or plant cell. It follows that such a genetically engineered plant, plant part, or plant cell has been prepared by technical means and does not occur in nature.

As can be taken from the appended examples it was surprisingly found that the photosynthesis of a plant, plant part, or plant cell can be increased by increasing the expression of the squamosa promoter binding protein-like gene 9 (SPL9 gene) in the plant, plant part, or plant cell and/or by increasing the level of the squamosa promoter binding protein-like protein 9 (SPL9 protein) in the plant, plant part, or plant cell. In more detail, in the examples a wild-type *C. hirsuta* strain 'Oxford' (Ox) was transformed with a rSPL9 construct. The rSPL9 construct comprises the *Arabidopsis thaliana* SPL9 promoter region in front of the SPL9 open reading frame fused to VENUS (i.e. a fluorescent protein derived from *Aequorea victoria),* followed by mutation of the miR156 binding site. Due to the mutation of the miR156 binding site the rSPL9 is protected from degradation by miR156 thereby increasing the mRNA and protein level of SPL9. It was found that increased *SPL9* correlates with higher photosynthetic rates in the rSPL9 *C. hirsuta* strain (Example 1.1). Example 1.3 further shows that the increased photosynthesis seen in rSPL9 *C. hirsuta* plants can be transferred to crop plants. As a proof of principle it is demonstrated that also in *rSPL9 Brassica napus* the increased *SPL9* correlates with higher photosynthetic rates. This shows the broad applicability of the method of the invention to plants in general and in particular to crop plants. This also shows that the *Arabidopsis thaliana* rSPL9 gene works in a cross-species manner not only in *C. hirsuta* but likewise in *Brassica napus.*

In accordance with a preferred embodiment of the first aspect the method comprises introducing into the plant, plant part, or plant cell a microRNA resistant mutant of a SPL9 gene, such that the microRNA resistant mutant of a SPL9 gene is expressed in the plant, plant part, or plant cell.

As explained above several *SPL* transcripts including *SPL9* can be degraded by miRNAs. The miRNAs bind to a miRNA Responsive Element (MRE) of the *SPL9* gene / mRNA that lies downstream of the conserved SBP domain. By mutating the MRE of the *SPL9* gene so that the miRNAs no longer bind generates a microRNA resistant mutant of a *SPL9* gene.

The SBP domain is a sequence specific DNA-binding domain found in plant proteins. Members of the SBP domain protein family probably function as transcription factors involved in the control of early flower development. They share a highly conserved DNA-binding domain that contains two zinc-binding sites. Among the 11 possible ligands for the zinc atoms that are conserved in the SBP zinc finger, only 8 are used. The SBP zinc finger follows the general pattern C-x4-C-x16-C-x2-[HC]-x15-C-x2-C-x3-H-x11-C. Three other histidines are well conserved but not involved in zinc binding; see InterPro data base entry IPR004333.

Since the MRE lies downstream of the conserved SBP domain the MRE can be found and identified in the SPL9 genes of essentially all kinds of plants by routine means.

In accordance with a more preferred embodiment of the first aspect the microRNA resistant mutant of a SPL9 gene comprises a mutated miR156 binding motif to which miRNA156 cannot bind or to which miRNA156 binds less.

In accordance with an even more preferred embodiment of the first aspect the non-mutated miR156 binding motif has the nucleotide sequence of SEQ ID NO: 1 (GTGCTCTCTCTCTTCTGTCA).

As discussed above, it is known that the microRNA156 (miR156) targets a series of SPL genes, including the SPL9 gene. The MRE of the *C. hirsuta* SPL9 gene, i.e. of the non-mutated miR156 binding motif is GTGCTCTCTCTCTTCTGTCA (SEQ ID NO: 1) (noting that the MRE of the *C. hirsuta* SPL9 gene is covered in *Arabidopsis thaliana*)*.*

It is a matter of routine to mutate this miR156 binding motif, so that miR156 can no longer bind or binds less. "Binding less" means in this context with increasing preference that the binding between miR156 and the mutated miR156 binding motif is 10x, 50x, 100x and 1000x weaker than between the miR156 and the non-mutated miR156 binding motif

The potential for miRNA:target site binding can be evaluated using either co-folding free energy measures or heuristic approaches, based on the identification of binding 'seeds', i.e., continuous stretches of binding corresponding to specific parts of the miRNA; see Klimentova et al. (2022), Genes (Basel); 13(12): 2323.

In accordance with another even more preferred embodiment of the first aspect the mutated miR156 binding motif has the nucleotide sequence of SEQ ID NO: 2 (GCGCATTGAGCTTGTTAAGC) or a sequence being at least 80% identical, preferably at least 85% identical, more preferably at least 90% identical and most preferably at least 95% identical thereto.

The mutated miR156 binding motif of GCGCATTGAGCTTGTTAAGC (SEQ ID NO: 2) has been prepared in the examples and miR156 can no longer bind to this mutated MRE. GCGCATTGAGCTTGTTAAGC (SEQ ID NO: 2) is part of *rSPL9* and the exact sequence of *rSPL9* will be further discussed herein below.

Herein above and also herein below sequence identities of at least 80% are envisioned. For each occurrence individually the at least 80% identity is with increasing preference at least 90%, at least 95%, at least 98%, and at least 99% identity. Means and methods for determining sequence identity are known in the art. Preferably, the BLAST (Basic Local Alignment Search Tool) program is used for determining the sequence identities as referred to herein.

In accordance with a preferred embodiment of the first aspect the microRNA resistant mutant of a *SPL9* gene comprises a sequence being at least 80% identical, preferably at least 85% identical, more preferably at least 90% identical and most preferably at least 95% identical to any one of SEQ ID NOs 3 to 8, provided that the mutated miR156 binding motif as comprised in any one of SEQ ID NOs 3 to 8 has the nucleotide sequence of SEQ ID NO: 2 (GCGCATTGAGCTTGTTAAGC), or a sequence being at least 80% identical, preferably at least 85% identical, more preferably at least 90% identical and most preferably at least 95% identical thereto.

SEQ ID NOs 3 to 8 are the nucleotide sequences of the *SPL9* genes of *Arabidopsis thaliana, Cardamine hirsuta* and *Brassica napus,* respectively. *Brassica napus* has four *SPL9* genes (SEQ ID NOs 5 to 8) because *Brassica napus* has a digenomic amphidiploid genome due to the interspecific hybridization between *B*. *oleracea* and *B. rapa.* Among SEQ ID NOs 3 to 8 SEQ ID NO: 3 is preferred because the SPL9 gene of *Arabidopsis thaliana* forms the basis of *rSPL9.*

While the method of the first aspect is expected to work for any SPL9 gene from any plant the *SPL9* genes of *Arabidopsis thaliana, Cardamine hirsuta* and *Brassica napus* are preferred.

*A. thaliana* is the most widely used model plant for studying plant sciences, including genetics, evolution, population genetics, and plant development and is therefore preferred. *Cardamine hirsuta* and *Brassica napus* are preferred because they are used in the appended examples.

In accordance with another preferred embodiment of the first aspect introducing comprises transforming, preferably stably transforming the plant, plant part, or plant cell with the microRNA resistant mutant of a SPL9 gene.

"Transformation" is used herein to describe the insertion of new genetic material into plant cells. There are two types of plant transformation: Stable transformation and transient transformation. Stable transformation is preferred.

Stable transformation refers to the stable introduction of a gene into a plant, plant part of plant cell; meaning that the gene will be fully integrated into the host genome, so that it is expressed continuously, and will also be expressed in later generations of the plant.

As explained above, in the examples herein below *C. hirsuta* strain and *Brassica napus* were stably transformed with the *rSPL9* construct. The sequence of the *rSPL9* construct is derived from *Arabidopsis thaliana* and comprises
(i) *Arabidopsis thaliana SPL9_*promoter (italic)
(ii) *Arabidopsis_thaliana_*SPL9_5'UTR (bold)
(iii) *Arabidopsis thaliana SPL9 ORF_*part 2 (underlined)
(iv) *Arabidopsis_thaliana_*SPL9_mutated_miR156_binding_motif (SEQ ID NO: 2)
(v) *Arabidopsis_thaliana_*SPL9_ORF_part 2 (underlined)
(vi) linker (double underlined)
(vii) VENUS (dotted underlined)

ctggcgaaagggggatgtgctgcaaggcgattaagttgggtaacgccagggttttcccagtcacgacgttgtaaaacgacggccagtgccaa
gctGGCCGCTCTAGAACTAGTGGatctcgagatttctaatgagtgtgaagatgctataattgcaaattgtggaacttaggcaaaata
taaaacagcaaagcccttaaagggaagagaactaagaagtgagaaaggagtaattagactttagagattagtctaaattgaaaagttaaaa
caaaattaaaattgtcaaccaatattaaaactttacaatagtaaatttaagatcttttcattgaaaaatgtcaaatagttatacatctccaacttaacc
aataaccacagatccaaaacccaaatttgccaccatttatatatatatatatatatatacatataactaataataagaaatttagcaagtatttgata
gtatttgcatcagcataaatcaaaattttctcaaaatgtctgtctagctatttcagaacctagacgtagttgtaaaactagtcaggggttaggagatc
gatcaggtctttagtttcagggttaaaccaatgcagccaaacaagtggaaaccactttcactaaataaagctaaacaaaaagaacaaatcaa
aacctaaacctataaaataatactatactacagtttacacagactctgatggatgatgtctatgactccgatttaacttttcatacttttcattataacga
atagatctttctttagatattgtataggtacaaattatcaaaaataaaaaaaagaagtccaaaaataaaactaatttggctttaactagcaaagag
aaacaaacaaactcaaaatcaagataataattagaagaagaaaaaacccatttttgtatatttaaacatgagagaaagaggtttaacaaataa
aaataagagtactgaattcatttatagggttgcttgcacatctgccgacaccaactagttcccttgaagtgtcacatcttttgttgtgttccaataactgt
actgctcatatttcagtctttaatacttttaaccttttcacgatcagtataaaaaaatttcttatcatattcgtcgttactctttaactaactacagtatagaa
gaagaaactacctatttatagaactctgatctcctcggcagtttcctattggttaccactgctgagtatatagaatctatggagaatagtatttttaaatt
tagaatgttactattaacttaagtacaaatcaatatcagattatcaaaattggtatagttggatgttctatggctatggggagtctgaatattgctccct
ggtctcagatagatgctttcaaaatttaatactacttaaatttaaacatacatcgccacatgacccatcactatcacggttacttatggatatccaac
attaaagagtaccacccaacactaggactcttataaaacacataaactccaaaattttatacctaatatccctggacaatatcaataaagttttatt
ggtttcttttttgcaacaaacgcaagagaaatttttattcgtcacggattgaggttttacgcacaccggtacaaaagtgtctatgaagaaaataaca

aaccatgcaaacgtgtttaacgggctttacaaatatgttcagaaattactcgcatgcgagattaaagaaatacaaaaaacaagtttggaaccgg
aggattgcaattatcatctattctatatcgcgtgtgtgtgtgtgtaaaattatgatttataatgtatcgaggtcttagattttaccttgcgatcctagagtttc
gtacatatatatcgactattcaagttcttctttcatctttttgtatcttaataagttccacaaatttcgacgatagagaccatacatttgttgttatcctctact
cacgaagacaaccagtctaacgttattgcataagttccacgtaaattaaaattgtgtttcttttgttaatattgaaaataggttaaaatgtcgccaaaa
aataagctttgaaaacttgaggtaggaaaaaatcatggttcttttgcacatgctccggaccaatcatgtttactactaaaattcttactgcttttataat
aatcatctcgactggcgcgtgtagaactctagatgaatgccaagtcttattttctaattataaacgcaaataataaaaaggtttaagaatatttaaat
ttcacatgacaatatgacacaataaatactacttgaaaaatgatctttgaaatttcatattttctaatcaaaaacaattccttcatattttgaaatataat
cgatctctcatatttaatttccaattagagaattatatccacatgatttgctttcaaatatgtctatttaacgtgtacgttgaacttgaacctatttattattta
attcattcccaaactaaaaatattttattttctaaaaaatatagtcgtaagcgtacttgtgtaaatatgttctaaagttaataatgggtttagacatttagt
agaaatatatattttttggtttttggtttggttggttcggataatcaaaattttcagtattcgactaaaatacatttttgagttatatttctttacttaattttcata
attgtaagtatttaaccttgtgtacgattaaaaaaaaaaaagtattttaaccttgtgtatcattcataatgtaggttttctttccattttgaaaaaaagttttg
atattggttcgcattcatcagttgaaaaccaagtcaaatttatggtagattggcaatggacaaacacaaaatatcatctccatttattgccaacctca
taatgccaaatggtggaccaaaaggaaaaacatgcatttatttattctcttgacataagagaaaatgcatttaacttgtcaaataattgcaagga
aaaactctataaaaagatagaaatgtgaacagtataaagtaaataaagaaatatgtgtacatgaaagacctctccaatgttcacttgct
ccagtttctccaagattagaaactgctacgtctctctctatagctccctcgttttgctatgtggtaattaacaacagacaacagtttttgttt
gtccgtaccgttttacttactctctcactgtctttgtctattaccactctcgtctctttcttttttccttctgttctgtttctctctctaaacccaaaa
cagtcaaaatcagggaagccgaaattttctttgctttcttctcctttggtcctttctttaaacccgagacagttaggtttgtgtgagagaga
gaatgatgagtaaaaccctttctgtctgagtaagaggaaaccaacGGTACCATGGAGATGGGTTCCAACTCGGGTC
CGGGTCATGGTCCGGGTCAGGCAGAGTCGGGTGGTTCCTCCACTGAGTCATCCTCTTTCAGTGG
AGGGCTCATGTTTGGCCAGAAGATCTACTTCGAGGACGGTGGTGGTGGATCCGGGTCTTCTTCC
TCAGGTGGTCGTTCAAACAGACGTGTCCGTGGAGGCGGGTCGGGTCAGTCGGGTCAGATACCA
AGGTGCCAAGTGGAAGGTTGTGGGATGGATCTAACCAATGCAAAAGGTTATTACTCGAGACACC
GAGTTTGTGGAGTGCACTCTAAAACACCTAAAGTCACTGTGGCTGGTATCGAACAGAGGTTTTGT
CAACAGTGCAGCAGgttcttttattcttcttcccagttctaccctttagttctattctttattaacatgttttgccattgccggagatattcgagtccg
gccaaggctctgcttgttccactgttgtgaatcaaatctaaaggctttagctttttgtgattgtcagtgtcatatgcttttttggctttaacgtctttttattttca
ttttttaaaaaactttacagcttagttttgtaacctggtcttgtgtttgttggactcgttgggctcatttttgggaatatgtttacgtctacttgtagatgcttctct
tttatttttcttttgattttgtctttctaaatatctaatccaaagagatatgaaatgaatggacaagaagaaagccgttgccctcatttttgcttctttaggtc
atatcattattgagggggacaaaactagtgtaccatagcatcattgacgtgatcattgaggaactctgttacattatattacaagtgtgtggttttaga
ttttaattcaaaactgatgacaaaagcttataatgactgatagaatgtatacaaacggtagttaacgtggagaggtcttgtctttggaatgttctgttat
gttgcttttctttgaagttaactcaatctgaatcaatcacatagGTTTCATCAGCTTCCGGAATTTGACCTAGAGAAAAGG
AGTTGCCGCAGGAGACTCGCTGGTCATAATGAGCGACGAAGGAAGCCACAGCCTGCGTCTCTCT
CTGTGTTAGCTTCTCGTTACGGGAGGATCGCACCTTCGCTTTACGgttagatccttaagctcgacacataagac
cgattcatgtacttttcatccttaaaagatagatgtatatgctacagAAAATGGTGATGCTGGAATGAATGGAAGCTTTCTT
GGGAACCAAGAGATAGGATGGCCAAGTTCAAGAACATTGGATACAAGAGTGATGAGGCGGCCAG
TGTCGTCACCGTCATGGCAGATCAATCCAATGAATGTATTTAGTCAAGGTTCAGTTGGTGGAGGA
GGGACAAGCTTCTCATCTCCAGAGATTATGGACACTAAACTAGAGAGCTACAAGGGAATTGGCGA
CTCAAACTGCGCATTGAGCTTGTTAAGCAATCCACATCAACCACATGACAACAACAACAACAACAA
CAACAACAACAACAACAACAACAATACATGGCGAGCTTCTTCAGGTTTTGGCCCGATGACGGTTA
CAATGGCTCAACCACCACCTGCACCTAGCCAGCATCAGTATCTGAACCCGCCTTGGGTATTCAAG
GACAATGATAATGATATGTCTCCTGTTTTGAATTTAGGTCGATACACCGAGCCAGATAATTGTCAG
ATAAGTAGTGGCACGGCAATGGGTGAGTTCGAGTTATCTGATCACCATCATCAAAGTAGGAGACA

GTACATGGAAGATGAGAACACAAGGGCTTATGACTCTTCTTCTCACCATACCAACTGGTCTCTCG
CTGCGGCTGCTGCCGCTGCGGCAGCGATGAGCAAGGGCGAGGAGCTGTTCACCGGGGTGGTG
CCCATCCTGGTCGAGCTGGACGGCGACGTAAACGGCCACAAGTTCAGCGTGTCCGGCGAGGGC
GAGGGCGATGCCACCTACGGCAAGCTGACCCTGAAGCTGATCTGCACCACCGGCAAGCTGCCC
GTGCCCTGGCCCACCCTCGTGACCACCCTGGGCTACGGCCTGCAGTGCTTCGCCCGCTACCCC
GACCACATGAAGCAGCACGACTTCTTCAAGTCCGCCATGCCCGAAGGCTACGTCCAGGAGCGCA
CCATCTTCTTCAAGGACGACGGCAACTACAAGACCCGCGCCGAGGTGAAGTTCGAGGGCGACAC
CCTGGTGAACCGCATCGAGCTGAAGGGCATCGACTTCAAGGAGGACGGCAACATCCTGGGGCA
CAAGCTGGAGTACAACTACAACAGCCACAACGTCTATATCACCGCCGACAAGCAGAAGAACGGC
ATCAAGGCCAACTTCAAGATCCGCCACAACATCGAGGACGGCGGCGTGCAGCTCGCCGACCACT
ACCAGCAGAACACCCCCATCGGCGACGGCCCCGTGCTGCTGCCCGACAACCACTACCTGAGCT
ACCAGTCCGCCCTGAGCAAAGACCCCAACGAGAAGCGCGATCACATGGTCCTGCTGGAGTTCGT
GACCGCCGCCGGGATCACTCTCGGCATGGACGAGCTGTACAAGTAA (SEQ ID NO: 15)

As discussed above, VENUS is a fluorescent protein derived from Aequorea Victoria and in the *rSPL* gene a nucleotide sequence encoding a peptide linker is used in order to link the VENUS encoding sequence to the SPL9 gene that comprises a mutated miR156 binding motif to which miRNA156 cannot bind and the promoter.

The linker and VENUS are only present to make the plant cell, plant part or plant cells that express rSPL detectable by microscopy. It follows that while it is most preferred to use rSPL for the transformation also only a sequence comprising only parts (i) to (v) can be used,

In accordance with a further preferred embodiment of the first aspect the plant part is a leaf, leaf stalk, root, seed, stem, flower or fruit.

Among this list of plant parts a leaf or a seed are preferred. As can be taken form the appended examples increasing SPL9 in plants also results in increased leaf complexity or heteroblastic rate and/or displays an increased seed mass.

In accordance with a preferred embodiment of the first aspect the plant is a crop plant or a tree, like birch and poplar.

In accordance with a more preferred embodiment of the first aspect the crop plant is selected from rapeseed, millet, corn, wheat, buckwheat, sorghum, quinoa, barley, rice, oat, proso, rye, sugarcane, sugar beet, sunflower, vegetables, fruits and trees (like birch and poplar).

As discussed above a plant, plant part, or plant cell displaying an increased photosynthesis is display an increased capacity to transform CO₂ to O₂, thereby aiding in combating global warming. Agricultural land covered a total of around 4.8 billion hectares on earth in 2021. Of this, around 1.6 billion hectares were agricultural land on which crop plants, for example, grain or animal feed was cultivated. If a substantial part of the crop plants has an increased capacity to transform CO₂ to O₂, this would significantly aid in combating global warming. In addition, crop plants with increased photosynthesis are useful for increasing crop productivity; see, for example, Ort et al. (2015), PNAS. 112:8529-8536 and Tholen et al. (2012), Plant Sci, 197:92-101. It follows that crop plants that can be obtained by the method of the invention confer the additional technical advantage of crop improvement (increased productivity) which is desirable in terms of global food security.

In accordance with a preferred embodiment of the first aspect the microRNA resistant mutant of a SPL9 gene, preferably a crucifer SPL9 gene is operably linked to a promoter, preferably a SPL9 promoter region and most preferably a SPL9 promoter region of any one of SEQ ID NOs 9 to 14.

The SPL9 gene as referred to herein is generally preferably a crucifer SPL9 gene and more preferably a cruciferous vegetable SPL9 gene. Accordingly, also the plant, plant part of plant cell is preferably a crucifer plant, plant part or plant cell and more preferably a cruciferous vegetable plant, plant part or plant cell.

Cruciferous vegetables are vegetables of the family Brassicaceae (also called Cruciferae) with many genera, species, and cultivars being raised for food production such as cauliflower, cabbage, kale, garden cress, bok choy, broccoli, Brussels sprouts, mustard plant and similar green leaf vegetables. The family takes its alternative name (Cruciferae, Neo-Latin for "cross-bearing") from the shape of their flowers, whose four petals resemble a cross.

*Arabidopsis thaliana, Cardamine hirsuta* and *Brassica napus* are all crucifers.

The SPL9 promoter region of any one of SEQ ID NOs 9 to 14 are the promoter regions of the SPL9 genes of *Arabidopsis thaliana, Cardamine hirsuta* and *Brassica napus,* respectively. *Brassica napus* has four SPL9 promoter regions (SEQ ID NOs 11 to 14) because *Brassica napus* has a digenomic amphidiploid genome due to the interspecific hybridization between *B*. *oleracea* and *B. rapa.* Among SEQ ID NOs 9 to 14 SEQ ID NO: 9 is preferred because the SPL9 promoter region of *Arabidopsis thaliana* is comprised in rSPL9 as used in the appended examples.

In accordance with a more preferred embodiment of the first aspect the introducing of the microRNA resistant mutant of a *SPL9* gene is via bacterial-mediated transformation, particle bombardment transformation, calcium-phosphate-mediated transformation, cyclodextrin-mediated transformation, electroporation, liposome-mediated transformation, nanoparticle-mediated transformation, polymer-mediated transformation, virus-mediated nucleic acid delivery, whisker-mediated nucleic acid delivery, microinjection, sonication, infiltration, polyethylene glycol-mediated transformation, or a combination thereof.

As mentioned, transformation is used herein to describe the insertion of new genetic material into plant cells. The transformation can be achieved by a number of available techniques and in particular by any one of bacterial-mediated transformation, particle bombardment transformation, calcium-phosphate-mediated transformation, cyclodextrin-mediated transformation, electroporation, liposome-mediated transformation, nanoparticle-mediated transformation, polymer-mediated transformation. Also virus-mediated nucleic acid delivery, whisker-mediated nucleic acid delivery, microinjection, sonication, infiltration, polyethylene glycol-mediated transformation can be used to introduce the microRNA resistant mutant of a SPL9 gene.

In accordance with a more preferred embodiment of the first aspect the plant displays an increased leaf complexity or heteroblastic rate and/or displays an increased seed mass.

As discussed above, increasing SPL9 in plants not only results in increased photosynthesis but also results in plants having an increased leaf complexity or heteroblastic rate, and/or plants that display an increased seed mass.

The purpose of the method of the first aspect "for increasing the photosynthesis of a plant, plant part, or plant cell" may therefore be substituted by or supplemented with one or both of the purposes "for increasing leaf complexity or heteroblastic rate of a plant, plant part, or plant cell" and "increasing seed mass of a plant, plant part, or plant cell". In this respect it is to be understood that the plant part does not necessarily and the plant cell does not display increased leaf complexity or heteroblastic rate and/or increased seed mass *per se* because the plant part is not necessarily and the plant cell is not a leaf or seed. However, the increased leaf complexity or heteroblastic rate and/or increased seed mass characterizes a whole plant that can be obtained from the plant part or plant cell.

The present invention relates in a second aspect to a plant, preferably a transgenic plant with increased photosynthesis, wherein the plant comprises, preferably in its genome, a microRNA resistant mutant of a SPL9 gene as defined in connection with the first aspect.

The present invention relates in a third aspect to a plant part or a plant tissue, preferably transgenic plant part or a plant tissue with increased photosynthesis, wherein the plant part or a plant tissue comprises, preferably in its genome, a microRNA resistant mutant of a SPL9 gene as defined in connection with the first aspect.

The definitions and preferred embodiments of the first embodiment of the invention apply *mutatis mutandis* to the second and third aspect of the invention.

The second and third aspects of the invention are directed to a plant, plant part or a plant tissue that can be obtained or, preferably, has been obtained by the method of the first aspect.

The plant, plant part, or plant tissue is preferably transgenic. The DNA of transgenic plants has been modified using genetic engineering techniques. The aim of genetic engineering techniques is generally to introduce a new trait to the plant which does not occur naturally in the species.

However, it also possible to produce a plant that comprises in its genome a microRNA resistant mutant of a SPL9 gene as defined in connection with the first aspect by random mutagenesis, for example, by physical agents such as x-ray and gamma-ray or chemicals such as ethyl-methane sulfonate (EMS). The high-throughput technique called TILLING (Targeting Induced Local Lesion IN Genomes) can then be used for identifying an individual mutant as generated by the genetic engineering techniques.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show:

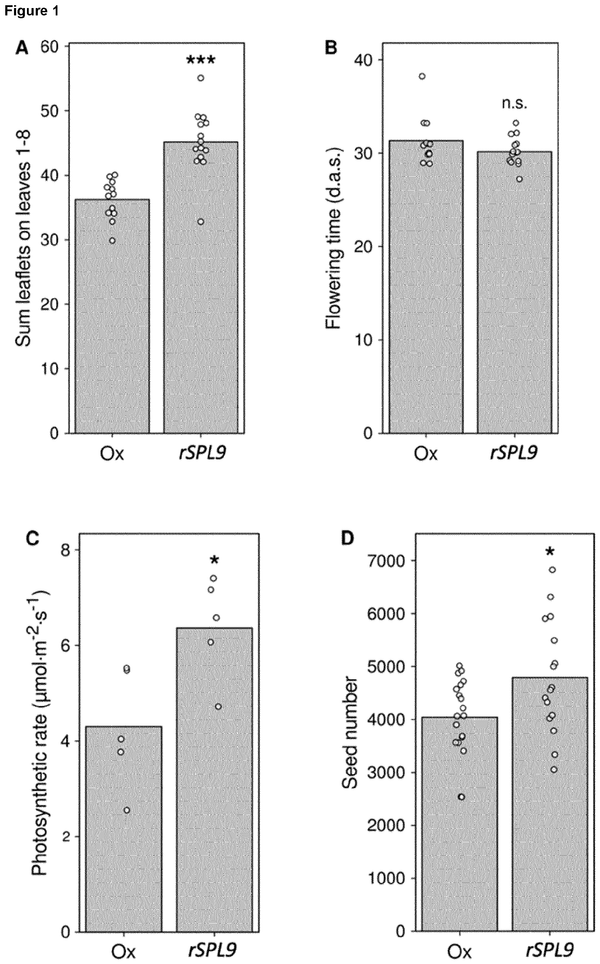

**Figure 1****. A miR156-resistant allele of SPL9 (rSPL9) positively affects leaflet number** (A), photosynthetic rate (C), and seed number (D) without affecting flowering time (B) in *Cardamine hirsuta.* Phenotypes of wild-type *C. hirsuta* Ox transformed with *rSPL9* were compared to wild-type control plants (Ox strain). Bars show mean trait values and points show individual observations. Differences between the means were tested with Student's t-test where * - p < 0.05, *** - p < 0.001, and n.s. - not significant. Ox, wild type; *rSPL9, rSPL9* allele, d.a.s, days after sowing.

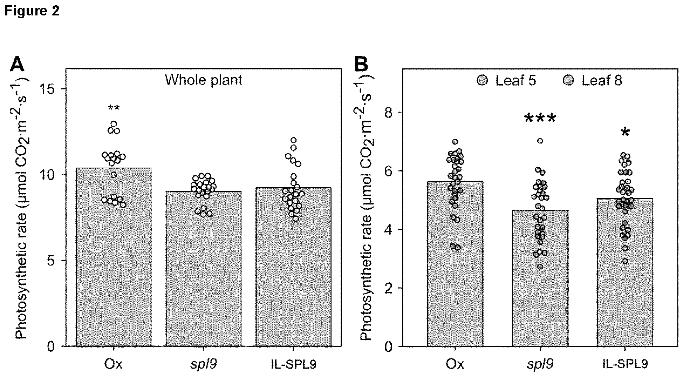

**Figure 2****. SPL9 controls photosynthetic rate in *Cardamine hirsuta.*** Photosynthetic rate was quantified as CO2 absorption using a LiCor LI-6800P photosynthesis system independently in whole plants (A) and in individual leaves (B). Bars show the mean photosynthetic rates and points show individual observations. In B, the colours indicate whether an observation was made on leaf 5 (cyan) or leaf 8 (magenta). Asterisks indicate significant differences according to mixed effects models that accounted for blocking (A and B) and leaf rank effects (B): * (P ≤ 0.05), ** (P ≤ 0.01), *** (P ≤ 0.001). In A, the asterisks at Ox mean both other genotypes have similarly significantly reduced photosynthetic rates, while in B, significance levels are shown for each genotype in comparison to Ox. Ox, wild type; spl9, spl9 mutant; IL-SPL9, Az1 allele.

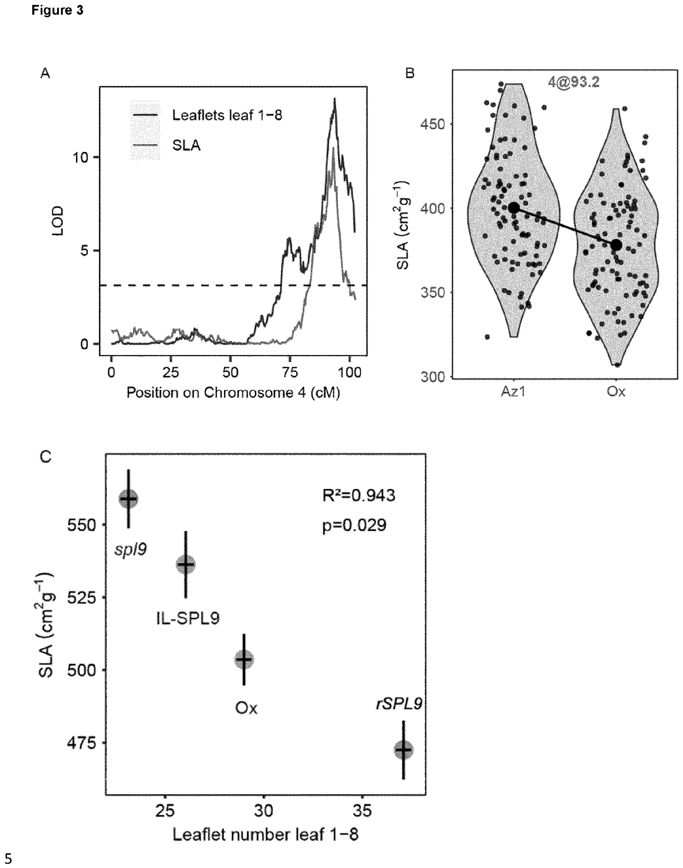

**Figure 3****. SPL9 contributes to** a **QTL for specific leaf area (SLA) in *Cardamine hirsuta.*** A) Co-locating QTL for leaflet number and SLA at the SPL9 locus on chromosome 4 in the Ox x Az1 RIL population. The LOD scores for the sum of the number of leaflets on leaves 1 through 8 (black) and SLA (green) are shown plotted against the genetic position in centi-Morgan (cM) on chromosome 4. The dotted horizontal line shows the significance threshold (α = 0.05). B) The additive allelic effect of the QTL shows that Az1 alleles confer higher SLA. The large black points show the mean SLA (y-axis) in RILs with the Az1 and Ox alleles respectively (x-axis) and the line indicates the allelic effect for a homozygous change from Az1 to Ox alleles. The small points show the mean SLA of individual RILs with some jitter applied along the x-axis, and the violins show the distribution of these points. C) A negative relationship between SLA (y-axis) and leaflet number (x-axis) in an SPL9 allelic series. Coloured circles show the mean SLA of the spl9 mutant, the introgression line (IL) with SPL9Az1 in the Ox background (IL-SPL9), the Ox strain, and the Ox transgenic line containing an miR156 resistant SPL9 (rSPL9); these constitute an allelic series from null through weak to strong, respectively. Vertical and horizontal black lines indicate the standard errors of the means. The correlation between SLA and leaflet number was significant (R²=0.943; P=0.029 Pearson's correlation). Ox, wild type; Az1, wild-type C. hirsuta `Azores1' strain.

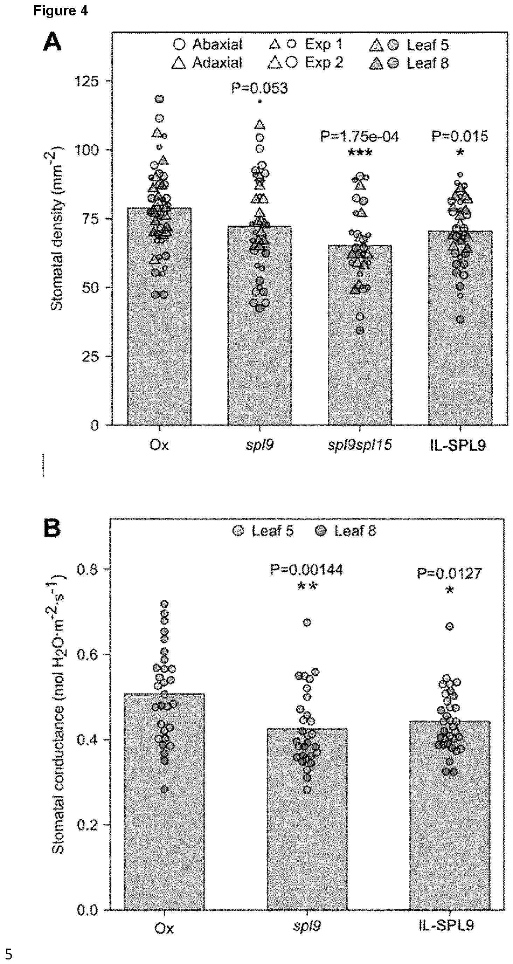

**Figure 4****. SPL positively affects stomatal density and stomatal conductance in *Cardamine hirsuta.*** A) Stomatal densities in the Ox strain, the *spl9* mutant, an *spl9spl15* double mutant, and the *SPL9* near isogenic line (NIL) with Az1 alleles introgressed in the Ox background (IL-SPL9). Bars show mean stomatal density as the number of stomata per mm2. Triangles and circles show individual observations from the adaxial and abaxial surfaces of the leaf respectively, smaller and larger symbols indicate data from two different experiments, and colours indicate the rank of the leaf that was observed (cyan, leaf 5; magenta, leaf 8). Note that although the spl9 mutant had only marginally reduced stomatal density in these experiments, this was strongly reduced in the double mutant with its close paralogue (spl9spl15), supporting the role of SPL in this trait. B) Stomatal conductances in the Ox strain, the spl9 mutant, and the SPL9 NIL with Az1 alleles introgressed in the Ox background (IL-SPL9). Bars show the mean trait values and circles show individual observations where colours indicate whether the observation was made on leaf 5 (cyan) or leaf 8 (magenta). Asterisks and a point indicate statistical significance in contrast to Ox according to mixed effects models accounting for experiment (in A only), block, leaf, and leaf surface (in A only) effects: ■ (P ≤ 0.1), * (P ≤ 0.05), ** (P ≤ 0.01), *** (P ≤ 0.001). Ox, wild type.

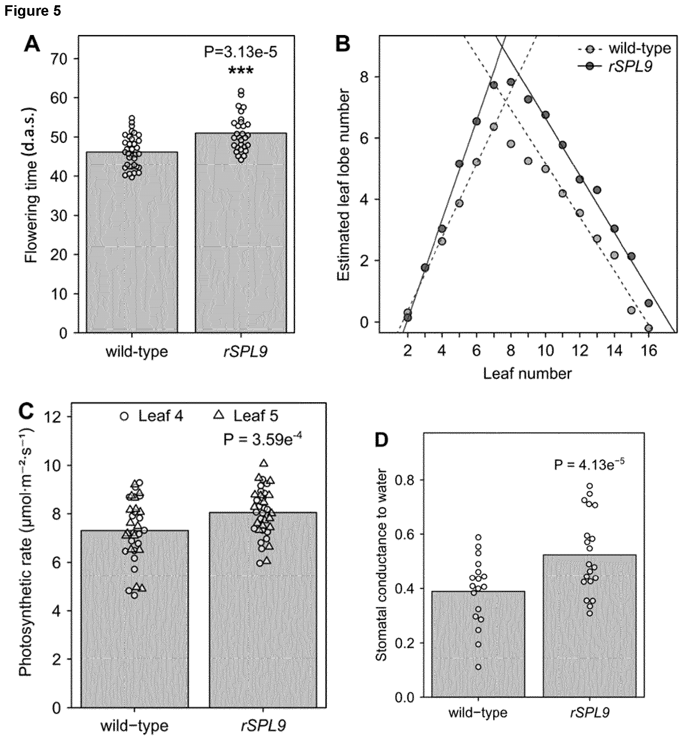

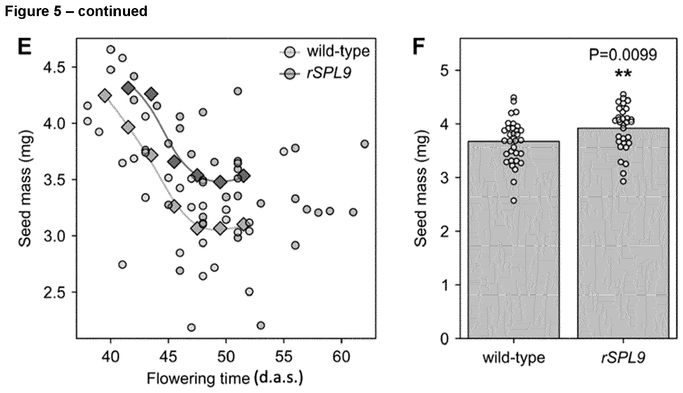

**Figure 5****. *rSPL9* affects heteroblastic progression, photosynthesis, and seed mass in Brassica napus.** A) *rSPL9* slightly increases flowering time in *B. napus.* Bars show the mean flowering time in days after sowing (d.a.s.) for the wild-type and *rSPL9* transgenic lines, and circles show individual observations. B) rSPL9 increases the rate of heteroblastic progression during the first phase (increasing), and also the maximum leaf complexity. Data from two independent experiments were analysed, and the estimated mean leaf lobe numbers while correcting for experiment, block, and line effects are shown plotted against the leaf rank (cyan, wild type; magenta, rSPL9). Heteroblastic rates were estimated independently in the phases of increasing and decreasing leaf complexity (dashed lines, wild type; solid lines, *rSPL9;* red, rate during increasing leaf lobe number; blue, rate during decreasing leaf lobe number). Note that although the rates are considerably different between the genotypes, the rank of the leaf where the maximum lobe number is reached (where the red and blue lines cross) is similar, indicating that *SPL9* specifically affects the rate of progression rather than the duration. C) Photosynthetic rates of wild-type B. napus Drakkar plants and the same line transformed with *rSPL9.* Bars show the mean photosynthetic rates estimated from a linear mixed-effects model while correcting for blocking, line, and leaf effects. Circles and triangles show the individual observations from leaves 4 and 5 respectively. D) Stomatal conductance to water in wild-type *B. napus* Drakkar plants and the same line transformed with *rSPL9.* Bars show the mean stomatal conductances while circles show individual observations. E) Seed mass is negatively correlated with residual flowering time in B. napus yet *rSPL9* consistently increases the former. Seed mass is plotted against flowering time for wild-type (cyan) and rSPL9 transgenic plants (magenta). Small circles show individual observations and large diamonds show mean seed mass in sliding windows along flowering time. Lines show smoothing splines through the sliding window means to reveal the underlying trend. F) *rSPL9* increases seed mass by 9.58% in B. napus. Bars show the mean seed mass (y-axis) in wild-type and rSPL9 transgenic *B. napus* Drakkar plants (x-axis), and circles show individual observations. All statistical tests consisted of linear mixed effects models that tested for the fixed effect of the transgene while accounting for line and block variance, P-values are shown in the figure panels and can also be indicated by asterisks where ** - P ≤ 0.01, *** P ≤ 0.001.

The examples illustrate the invention.

### Example 1 - Results

*SQUAMOSA PROMOTER BINDING PROTEIN-LIKE* 9 *(SPL9),* a paralogue of *SPL15* was identified as the gene underlying a quantitative trait locus (QTL) with heterochronic effects on leaflet heteroblasty in *Cardamine hirsuta* (Baumgarten, Pieper et al., 2023). Previous studies have documented critical and conserved roles for the SPL gene family in regulating many aspects of plant growth and development, such as: flowering time, leaf development, phase transition, plant architecture, organ size, fruit development, and stress response. Underlying many of these functions is a miR156-SPL9 regulatory module, which is a key factor in the control of plant aging (Wu et al., 2009). To investigate further the function of *SPL9* including its potential impacts on plant physiology, a CRISPR/*Cas9* mutant line for *SPL9* (hereafter referred to as *spl9*)*,* as well as a transgenic line expressing a dominant, miR156 resistant, allele of *SPL9* (hereafter referred to as *rSPL9*)*,* which can no longer be post-transcriptionally targeted for degradation by miR156 were produced thereby increasing the level of *SPL9* in *C*. *hirsuta,.* The *rSPL9* construct was made by cloning the *Arabidopsis thaliana SPL9* promoter region in front of the SPL9 open reading frame fused to VENUS, followed by mutation of the *miR156* binding site (Wang et al., 2008) by overlap extension PCR. The *rSPL9* line was produced by transforming the wild-type *C. hirsuta* strain 'Oxford' (Ox) with this *rSPL9* construct.

**Box 1:** Alignment of conserved region in *rSPL9* line and *A*. *thaliana SPL9* (*AtSPL9*)*,* showing modification of the *miR156* binding motif (positions 1466-1485 in *rSPL9*)*.* 
rSPL9 1441 TACAAGGAATTGGCGACTCAAACTGCGCATTGAGCTTGTTAAGCAATCCACATCAAC 1498
||||||||||||||||||||||||||| || | | | ||||||||||||
AtSPL9 1873 TACAAGGAATTGGCGACTCAAACTGTGCTCTCTCTCTTCTGTCAATCCACATCAAC 1930
SEQ ID NO: 1, non-mutated miR156 binding motif (GTGCTCTCTCTCTTCTGTCA)
SEQ ID NO: 2, mutated miR156 binding motif (GCGCATTGAGCTTGTTAAGC)

### Example 1.1: The effects of rSPL9 on development and physiology in Cardamine hirsuta

Having established suitable genetic material with altered SPL9 activity (*spl9,* reduced activity; *rSPL9,* increased activity), this was then used to test for effects on photosynthetic rates and seed yield. It was found that *rSPL9* greatly increased leaflet number on leaf nodes 1-8 (Figure 1A) without affecting flowering time (Figure 1B), and that this was correlated with higher photosynthetic rate (Figure 1C), and increased seed number (Figure 1D) when compared to the Ox control. These results underscore the potential of the heterochronic *SPL* pathway to specifically control leaf form and function, and seed yield.

This work was followed with an analysis of the *spl9* mutant alongside the naturally attenuated Az1 allele that had previously identified by the inventors in a population survey of *C. hirsuta* in the Azores archipelago (Baumgarten, Pieper et al., 2023). These assays revealed a decrease in the photosynthetic rates of whole plants (A) as well as of individual leaves (B) in the lines bearing either the *spl9* mutant or Az1 allele (Figure 2). Notably, the latter findings demonstrate a direct link between the complexity of individual leaves and their contribution to the photosynthetic rates of the entire plant.

### Example 1.2: Changes in specific leaf area and stomatal density in Cardamine hirsuta with altered SPL9 function

The above observations (Figures 1-2) demonstrate that SPL9 positively influences CO₂ fixation in *C. hirsuta.* They also raise the question of how altered SPL9 levels affect photosynthesis in *C. hirsuta.* To answer this, aspects of final leaf morphology were characterized that could underly the physiological changes in the leaf that are associated with altered levels of SPL9. A strong QTL for specific leaf area (SLA; i.e., leaf area per unit mass) was found in the Ox × Az1 recombinant inbred line (RIL) population, which co-located with the leaflet number QTL on chromosome 4 that the inventors had previously shown to be caused by SPL9 (Figure 3A-B; Baumgarten, Pieper et al., 2023). Moreover, this QTL was validated by characterising an *SPL9* allelic series in *C. hirsuta,* in which a strong negative relationship between SLA and SPL9 activity was found, as determined by its effect on leaflet number (Figure 3C). This provides evidence that higher SPL9 expression leads to denser leaves. According to current thinking, having more yet smaller mesophyll cells may have a positive effect on photosynthesis on a per area leaf basis - perhaps by increasing the surface area available for gas exchange (Lundgren and Flemming 2020; Lawrence et al., 2021).

In similar experiments, a positive relationship between stomatal density and SPL9 activity was also found (Figure 4A). All else being equal, more stomata can in principle increase CO₂ fixation by increasing stomatal conductance (Lundgren and Flemming, 2020). In agreement with their lower stomatal densities, the stomatal conductances of the *C*. *hirsuta spl9* mutant and the *SPL9Az1* introgression line (*IL-SPL9*) were significantly reduced (Figure 4B). Thus, because SLA and stomatal density can influence photosynthetic rate to a similar degree, they both offer a plausible explanation for how SPL9 can affect photosynthesis. This information also raises the possibility that tinkering with growth and proliferation pathways can provide a path for the coordinated modification of multiple anatomical traits (in this case SLA and stomatal density) that can improve photosynthetic physiology.

### Example 1.3: Characterisation of SPL9 in the crop Brassica Napus

To evaluate whether the increased photosynthesis seen in *C. hirsuta* plants with higher levels of SPL9 expression was transferrable to crop plants, *Brassica napus* lines were generated expressing modified SPL9. To this end, *rSPL9* was introduced into *B. napus* - the same allele used to transform *C. hirsuta* as described above. 63 independent transgenic lines were acquired, which allowed us to select an allelic series displaying from weak to strong effects on leaf heteroblasty. These materials were then used to test for correlated effects on photosynthesis and seed production.

A small yet significant increase in flowering time in *B. napus* plants transformed with *rSPL9* was found (Figure 5A). Notably, although in *C. hirsuta* flowering time and leaflet number tended to be negatively correlated (Cartolano et al., 2015), it was found that *B. napus* plants expressing rSPL9 had increased leaf complexity and an increased rate of heteroblastic progression (Figure 5B). The increased rate of heteroblastic progression towards the maximum leaf complexity was comparable to the effect of *rSPL9* in *C. hirsuta* (Figure 1A). Additionally, *B. napus rSPL9* plants showed increased photosynthetic rate and stomatal conductance (Figure 5C-D), as well as 9.58% increased seed mass (Figure 5E-F), which was consistent with similar correlated effects on seed mass and heteroblastic progression observed in *C. hirsuta* (Cartolano et al., 2015).

### Example 2 - Conclusion

When investigating the role of SPL9 in heteroblasty in our model species *C. hirsuta,* it was determined that reduced levels of SPL9 caused reduced rates of photosynthesis. This stimulated us to investigate the potential application of increasing photosynthesis in crops via tinkering with SPL9 levels. Increased leaf complexity and elevated photosynthesis were discovered in transgenic *B. napus* plants expressing a microRNA156 resistant version of *A*. *thaliana* SPL9. Furthermore, it was also demonstrated that altering SPL9 expression is able to increase photosynthesis, leaf heteroblastic rate, and seed mass in this crop species. These findings demonstrate that new information on the control of plant development and its impact upon physiology in model systems can be successfully transferred to crop species to address the need for novel strategies for increasing photosynthesis and yield. This approach of tinkering with SPL9 levels can also be used in strategies to use suitably modified plants to reduce atmospheric CO₂ to combat global warming. Notably, it was found that it is possible to increase CO₂ fixation in *B. napus* by expressing a dominant microRNA resistant *SPL9* allele. Such microRNA resistant alleles are highly attractive as breeding targets because they can be readily introduced in desired germplasm with new breeding technologies, they can be easily identified after EMS mutagenesis through TILLING, and they do not need to be homozygous to have an effect. Their dominant nature also allows them to be easily combined with other breeding targets. Finally, because it is possible to attain varying levels of SPL expression by introducing different mutations in the microRNA binding site, one can envisage fine-tuning SPL levels by testing multiple mutations that cause SPL to be insensitive to inhibition. This may allow the optimization of CO₂ fixation relative to other SPL9-regulated traits, and help prevent pleiotropic effects arising from altered SPL9 expression.

### References

Baumgarten L, Pieper B, Song B, Mane S, Lempe J, Lamb J, et al. (2023) Pan-European study of genotypes and phenotypes in the Arabidopsis relative Cardamine hirsuta reveals how adaptation, demography, and development shape diversity patterns. PLoS Biol 21(7): e3002191. doi.org/10.1371/journal.pbio.3002191
Cartolano M, Pieper B, Lempe J, Tattersall A, Huijser P, Tresch A, Darrah PR, Hay A, Tsiantis M. (2015). Heterochrony underpins natural variation in Cardamine hirsuta leaf form. PNAS 112(33): 10539-44
Lawrence EH, Springer CJ, Helliker BR, Poethig RS. (2021) MicroRNA156-mediated changes in leaf composition lead to altered photosynthetic traits during vegetative phase change. New Phytol, 231: 1008-1022. https://doi.org/10.1111/nph.17007
Lundgren MR, Fleming AJ. (2020) Cellular perspectives for improving mesophyll conductance. Plant J, 101: 845-857. doi.org/10.1111/tpj.14656
Wu G, Park MY, Conway SR, Wang JW, Weigel D, Poethig RS. (2009) The sequential action of miR156 and miR172 regulates developmental timing in Arabidopsis. Cell 138(4):750-9. doi: 10.1016/j.cell.2009.06.031
Wang, J.W., Schwab, R., Czech, B., Mica, E., and Weigel, D. (2008). Dual effects of miR156-targeted SPL genes and CYP78A5/KLUH on plastochron length and organ size in Arabidopsis thaliana. Plant Cell 20, 1231-1243.

## Claims

1. A method for increasing the photosynthesis of a plant, plant part, or plant cell by increasing the expression of the squamosa promoter binding protein-like gene 9 (SPL9 gene) in the plant, plant part, or plant cell and/or by increasing the level of the squamosa promoter binding protein-like protein 9 (SPL9 protein) in the plant, plant part, or plant cell.

2. The method of claim 1 comprising introducing into the plant, plant part, or plant cell a microRNA resistant mutant of a SPL9 gene, such that the microRNA resistant mutant of a SPL9 gene is expressed in the plant, plant part, or plant cell.

3. The method of claim 2, wherein the microRNA resistant mutant of a SPL9 gene comprises a mutated miR156 binding motif to which miRNA156 cannot bind or to which miRNA156 binds less.

4. The method of claim 3, wherein the non-mutated miR156 binding motif has the nucleotide sequence of SEQ ID NO: 1 (GTGCTCTCTCTCTTCTGTCA).

5. The method of claim 3, wherein the mutated miR156 binding motif has the nucleotide sequence of SEQ ID NO: 2 (GCGCATTGAGCTTGTTAAGC) or a sequence being at least 80% identical, preferably at least 85% identical, more preferably at least 90% identical and most preferably at least 95% identical thereto.

6. The method of any one of claims 1 to 5, wherein the microRNA resistant mutant of a SPL9 gene comprises a sequence being at least 80% identical, preferably at least 85% identical, more preferably at least 90% identical and most preferably at least 95% identical to any one of SEQ ID NOs 3 to 8, provided that the mutated miR156 binding motif as comprised in any one of SEQ ID NOs 3 to 8 has the nucleotide sequence of SEQ ID NO: 2 (GCGCATTGAGCTTGTTAAGC), or a sequence being at least 80% identical, preferably at least 85% identical, more preferably at least 90% identical and most preferably at least 95% identical thereto.

7. The method of any one of claims 2 to 6, wherein introducing comprises transforming, preferably stably transforming the plant, plant part, or plant cell with the microRNA resistant mutant of a SPL9 gene.

8. The method of any one of claims 1 to 7, wherein the plant part is a leaf, leaf stalk, root, seed, stem, flower or fruit.

9. The method of any one of claims 1 to 9, wherein the plant is a crop plant.

10. The method of claim 9, wherein the crop plant is selected from rapeseed, millet, corn, wheat, buckwheat, sorghum, quinoa, barley, rice, oat, proso, rye, sugarcane, sugar beet, sunflower, vegetables, fruits and trees like birch and poplar.

11. The method of any one of claims 2 to 10, wherein the microRNA resistant mutant of a SPL9 gene, preferably a crucifer SPL9 gene is operably linked to a promoter, preferably a SPL9 promoter region and most preferably a SPL9 promoter region of any one of SEQ ID NOs 9 to 14.

12. The method of any one of claims 1 to 11, wherein the introducing is via bacterial-mediated transformation, particle bombardment transformation, calcium-phosphate-mediated transformation, cyclodextrin-mediated transformation, electroporation, liposome-mediated transformation, nanoparticle-mediated transformation, polymer-mediated transformation, virus-mediated nucleic acid delivery, whisker-mediated nucleic acid delivery, microinjection, sonication, infiltration, polyethylene glycol-mediated transformation, or a combination thereof.

13. The method of any one of claims 1 to 12, wherein the plant displays an increased leaf complexity heteroblastic rate and/or displays an increased seed mass.

14. A plant, preferably a transgenic plant with increased photosynthesis, wherein the plant comprises, preferably in its genome, a microRNA resistant mutant of a SPL9 gene as defined in any one of the preceding claims.

15. A plant part or a plant tissue, preferably a transgenic plant part or a plant tissue with increased photosynthesis, wherein the plant part or a plant tissue comprises, preferably in its genome, a microRNA resistant mutant of a SPL9 gene as defined in any one of the preceding claims.
